# NEW EUROPEAN PATENT SPECIFICATION

(11) **EP 1 301 135 B2**
(45) Date of publication and mention of the opposition decision: **18.08.2010**
(45) Mention of the grant of the patent: 07.09.2005
(21) Application number: 01923193.5
(22) Date of filing: 06.04.2001
(51) Int. Cl.: A61B 18/14

(54) **BIPOLAR ELECTROSURGICAL FORCEPS WITH NON-CONDUCTIVE STOP MEMBERS**
BIPOLARE ELEKTROCHIRURGISCHE ZANGE MIT NICHTLEITENDEN ABSTANDHALTERN
PINCES ELECTROCHIRURGICALES BIPOLAIRES AVEC ELEMENTS D'ARRET NON CONDUCTEURS

(30) Priority: 21.07.2000 US 621029
(43) Date of publication of application: 16.04.2003
(73) Proprietor: Covidien AG, 8212 Neuhausen am Rheinfall (CH)
(72) Inventor: DYCUS, Sean T., Superior, CO 80027 (US); BUYSSE, Steven, Paul, Longmont, CO 80501 (US); FRAZIER, Randel, Alven, Louisville, CO 80027 (US); BROWN, Dax, D., Vinta, OK 74301 (US)
(74) Representative: HOFFMANN EITLE
(86) International application number: PCT/US2001/011222
(87) International publication number: WO 2002/007627

(56) References cited:
- WO-A-00//24 330
- DE-A- 3 640 471
- US-A- 5 391 166
- US-A- 5 499 997
- 1983 Catalogue of Products from Mentor O&O, Inc.
- Mentor O&O, Inc., Ophthalmic diagnostic and surgical instruments, 1984

## Description

### BACKGROUND

The present disclosure relates to an electrosurgical instrument for performing open and/or endoscopic surgical procedures. More particularly, the present disclosure relates to a bipolar electrosurgical forceps which includes a non-conductive stop member associated with one or both of the opposing jaw members which is designed to control the gap distance between opposing jaw members and enhance the manipulation and gripping of tissue during the sealing process.

### Technical Field

A hemostat or forceps is a simple plier-like tool which uses mechanical action between its jaws to constrict vessels and is commonly used in open surgical procedures to grasp, dissect and/or clamp tissue. Electrosurgical forceps utilize both mechanical clamping action and electrical energy to effect hemostasis by heating the tissue and blood vessels to coagulate, cauterize and/or seal tissue.

Over the last several decades, more and more surgeons are complimenting traditional open methods of gaining access to vital organs and body cavities with endoscopes and endoscopic instruments which access organs through small puncture-like incisions. Endoscopic instruments are inserted into the patient through a cannula, or port, that has been made with a trocar. Typical sizes for cannulas range from three millimeters to twelve millimeters. Smaller cannulas are usually preferred, and this presents a design challenge to instrument manufacturers who must find ways to make surgical instruments that fit through the cannulas.

Certain surgical procedures require cutting blood vessels or vascular tissue. However, due to space limitations surgeons can have difficulty suturing vessels or performing other traditional methods of controlling bleeding, e.g., clamping and/or tying-off transected blood vessels. Blood vessels, in the range below two millimeters in diameter, can often be closed using standard electrosurgical techniques. If a larger vessel is severed, it may be necessary for the surgeon to convert the endoscopic procedure into an open-surgical procedure and thereby abandon the benefits of laparoscopy.

Several journal articles have disclosed methods for sealing small blood vessels using electrosurgery. An article entitled Studies on Coagulation and the Development of an Automatic Computerized Bipolar Coagulator, J. Neurosurg., Volume 75, July 1991, describes a bipolar coagulator which is used to seal small blood vessels. The article states that it is not possible to safely coagulate arteries with a diameter larger than 2 to 2.5 mm. A second article is entitled Automatically Controlled Bipolar Electrocoagulation - "COA-COMP", Neurosurg. Rev. (1984), pp.187-190, describes a method for terminating electrosurgical power to the vessel so that charring of the vessel walls can be avoided.

By utilizing an electrosurgical forceps, a surgeon can either cauterize, coagulate/desiccate and/or simply reduce or slow bleeding, by controlling the intensity, frequency and duration of the electrosurgical energy applied to the tissue. Generally, the electrical configuration of electrosurgical forceps can be categorized in two classifications: 1) monopolar electrosurgical forceps; and 2) bipolar electrosurgical forceps.

Monopolar forceps utilize one active electrode associated with the clamping end effector and a remote patient return electrode or pad which is typically attached externally to the patient. When the electrosurgical energy is applied, the energy travels from the active electrode, to the surgical site, through the patient and to the return electrode.

Bipolar electrosurgical forceps utilize two generally opposing electrodes which are disposed on the inner opposing surfaces of the end effectors and which are both electrically coupled to an electrosurgical generator. Each electrode is charged to a different electric potential. Since tissue is a conductor of electrical energy, when the effectors are utilized to grasp tissue therebetween, the electrical energy can be selectively transferred through the tissue.

In order to effect a proper seal with larger vessels, two predominant mechanical parameters must be accurately controlled - the pressure applied to the vessel and the gap between the electrodes both of which affect thickness of the sealed vessel. More particularly, accurate application of the pressure is important to oppose the walls of the vessel, to reduce the tissue impedance to a low enough value that allows enough electrosurgical energy through the tissue, to overcome the forces of expansion during tissue heating and to contribute to the end tissue thickness which is an indication of a good seal. It has been determined that a fused vessel wall is optimum between 0.001 and 0.005 inches. Below this range, the seal may shred or tear and above this range the lumens may not be properly or effectively sealed.

Electrosurgical methods may be able to seal larger vessels using an appropriate electrosurgical power curve, coupled with an instrument capable of applying a large closure force to the vessel walls. It is thought that the process of coagulating small vessels is fundamentally different than electrosurgical vessel sealing. For the purposes herein, "coagulation" is defined as a process of desiccating tissue wherein the tissue cells are ruptured and dried and vessel sealing is defined as the process of liquefying the collagen in the tissue so that it reforms into a fused mass. Thus, coagulation of small vessels is sufficient to permanently close them. Larger vessels need to be sealed to assure permanent closure.

Numerous bipolar electrosurgical forceps have been proposed in the past for various open surgical procedures. However, some of these designs may not provide uniformly reproducible pressure to the blood vessel and may result in an ineffective or non-uniform seal. For example, U.S. Patent No. 2,176,479 to Willis, U.S. Patent Nos. 4,005,714 and 4,031,898 to Hiltebrandt, U.S. Patent Nos. 5,827,274, 5,290,287 and 5,312,433 to Boebel et al., U.S. Patent Nos. 4,370,980, 4,552,143, 5,026,370 and 5,116,332 to Lottick, U.S. Patent No. 5,443,463 to Stem et al., U.S. Patent No. 5,484,436 to Eggers et al. and U.S. Patent No. 5,951,549 to Richardson et al., all relate to electrosurgical instruments for coagulating, cutting and/or sealing vessels or tissue. Furthermore, in US 5,499,997 an endoscopic tenaculum surgical instrument is disclosed. The endoscopic grasping tool is intended for use inside a patient and comprises a connector rod and first and second jaw arms, each arm having a tooth provided on the distal end thereof. By means of abutment between first and second contact pads, lateral displacement of the teeth is prevented.

US 5,391,166 describes bipolar electrosurgical endoscopic instruments having a detachable working end. The detachable working end includes movable severing or grasping members, e.g. scissors-like shearing members, and bipolar electrodes on opposing members for simultaneously severing tissue and causing hemostasis of the tissue. An electrically insulating layer is interposed between the movable members, in order to confine current flow to a region from where cutting edges of the movable members contact each other to a point distal to the cutting point.

These instruments rely an clamping pressure alone to procure proper sealing thickness and are not designed to take into account gap tolerances and/or parallelism and flatness requirements which are parameters which, if properly controlled, can assure a consistent and effective tissue seal. For example, it is known that it is difficult to adequately control thickness of the resulting sealed tissue by controlling clamping pressure alone for either of two reasons: 1) if too much force is applied, there is a possibility that the two poles will touch and energy will not be transferred through the tissue resulting in an ineffective seal; or 2) if too low a force is applied, a thicker less reliable seal is created.

As mentioned above, in order to properly and effectively seal larger vessels, a greater closure force between opposing jaw members is required. It is known that a large closure force between the jaws typically requires a large moment about the pivot for each jaw. This presents a challenge because the jaw members are typically affixed with pins which are positioned to have a small moment am-1s with respect to the pivot of each jaw member. A large force, coupled with a small moment arm, is undesirable because the large forces may shear the pins. Moreover and with particular respect to endoscopic procedures, it may also be undesirable to increase the moment arm of the pins because the physical size of the jaw members and other component parts might not fit through a cannula.

Moreover, increasing the closure forces between electrodes may have other undesirable effects, e.g., it may cause the opposing electrodes to come into close contact with one another which may result in a short circuit and a small closure force may cause pre-mature movement of the issue during compression and prior to activation.

Thus, a need exists to develop a bipolar forceps which effectively seals vascular tissue and solves the aforementioned problems by providing an instrument which produces a large closure force between the opposing jaws members, reduces the chances of short circuiting the opposing jaws during activation and assists in manipulating, gripping and holding the tissue prior to and during activation.

### SUMMARY

Aspects of the invention are defined in claims 1 and 2 below.

The present disclosure relates to a bipolar forceps for clamping and sealing tissue for use in open or endoscopic surgical procedures. The forceps includes at least one elongated shaft having opposing jaw members at a distal end thereof. The jaw members are movable relative to one another from a first position wherein the jaw members are disposed in spaced relation relative to one another to a second position wherein the jaw members cooperate to grasp tissue therebetween. The forceps connect to a source of electrical energy which, in turn, connects to each jaw member such that the jaw members are capable of conducting energy through tissue held therebetween. A raised lip acts as a stop member which projects from the inner-facing surface and extends about the outer periphery of the jaw member to control the gap distance between jaw members. In another embodiment, a longitudinally-oriented ridge extends from the proximal end to the distal end of one of the jaw members and controls the gap distance between the jaw members.

The stop members are affixed/attached to the jaw member(s) by stamping, thermal spraying, overmolding and/or by an adhesive. Preferably, the stop members project about 25.4 µm (about 0.001 inches) to about 127 µm (about 0.005 inches) and, preferably, about 50.8 µm (about 0.002 inches) to about 76.2 µm (about 0.003 inches) from the inner-facing surface of at least one of the jaw members. It is envisioned that the stop members may be made from an insulative material such as parylene, nylon and/or ceramic.

A bipolar forceps is disclosed having a drive rod assembly which electrically connects the jaw members to the source of electrical energy such that the first jaw member has a first electrical potential and the second jaw member has a second electrical potential. A handle may be attached to the drive rod assembly for imparting movement of the first and second jaw members relative to one another from the first and second positions.

In addition, a bipolar forceps is shown, which includes a pair of elongated shafts each having a jaw member at a distal end thereof and a finger ring at a proximal end thereof. Movement of the finger rings imparts movement of the jaw members relative to one another from the first position wherein the jaw members are disposed in spaced relation relative to one another to a second position wherein the jaw members cooperate to grasp tissue therebetween. The first shaft connects to the source of electrical energy to supply the first jaw member to a first electrical potential and the second shaft connects the second jaw member to a second electrical potential such that the jaw members are capable of conducting energy through the tissue held therebetween.

### BRIEF DESCRIPTION OF THE DRAWINGS

Various embodiments of the subject instrument are described herein with reference to the drawings wherein:
Fig. 1 is a perspective view of an endoscopic forceps;
Fig. 2 is an enlarged, perspective view of an end effector assembly of the forceps of Fig. 1;
Fig. 3 is a perspective view with parts separated of a handle assembly and activator of the forceps of Fig. 1;
Fig. 4 is an enlarged, perspective view with parts separated of the end effector assembly and a drive rod assembly of the forceps of Fig. 1;
Fig. 5A is a side, partial cross-section of the handle assembly and drive rod assembly of the forceps of Fig.1;
Fig. 5B is an enlarged, side cross-section of the indicated area of detail shown in Fig. 5A;
FIG. 6 is a perspective view of the handle assembly, activator and drive rod assembly of the forceps of Fig. 1;
Fig. 7 is an enlarged, partial cross-section of the end effector assembly shown with a pair of jaw members in the open configuration;
Fig. 8 is an enlarged, partial cross-section showing the linear motion of the drive rod assembly against a cam follower of the end effector assembly to effect closure of the jaw members;
Fig. 9 is a perspective view of the forceps showing the rotational movement of a rotating assembly which rotates the end effector assembly about a longitudinal axis "A";
Fig. 10 is an enlarged perspective view of the indicated area of detail shown in Fig. 9;
Fig. 11 is a perspective view of the forceps of the present disclosure shown sealing a tubular vessel through a cannula assembly;
Fig. 12 is an enlarged perspective view of a sealing site of a tubular vessel;
Fig. 13 is a longitudinal cross-section of the sealing site taken along line 13-13 of Fig. 12;
Fig. 14 is a longitudinal cross-section of the sealing site of Fig. 12 after separation of the tubular vessel;
Fig. 15A is a perspective view of an open forceps according to the present disclosure;
Fig. 15B is an enlarged view of the forceps of Fig. 15A; and
Figs. 16A-16G of which Figs. 16A to 16G only Figures 16A and 16E are embodiments of the claimed invention, are enlarged, perspective views showing alternative embodiments of a non-conductive stop member disposed on or along the inner-facing surface of one of the jaw members.

### DETAILED DESCRIPTION OF THE PREFERRED EMBODIMENTS

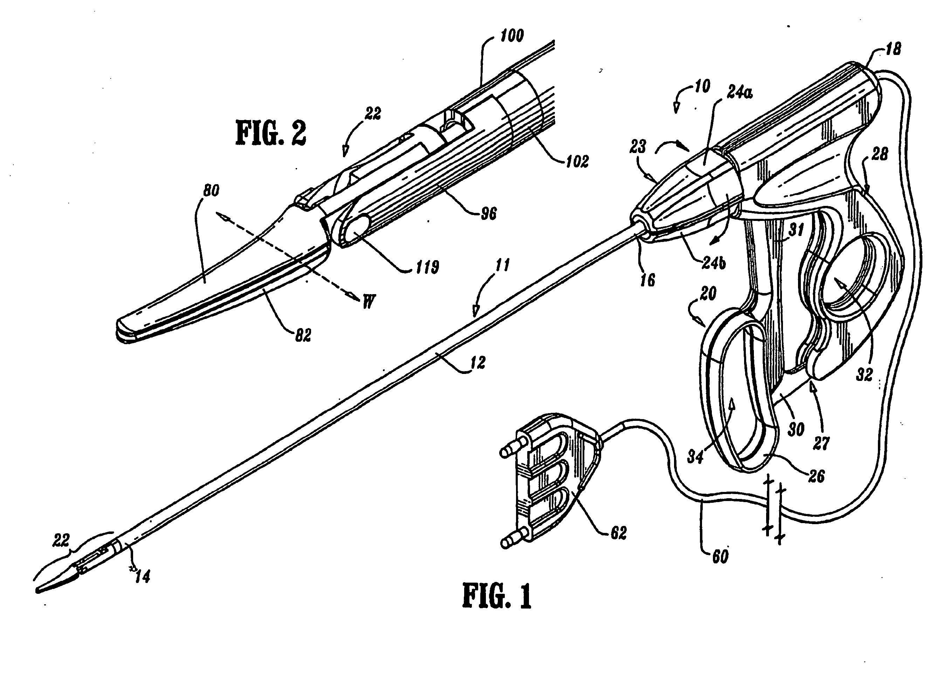

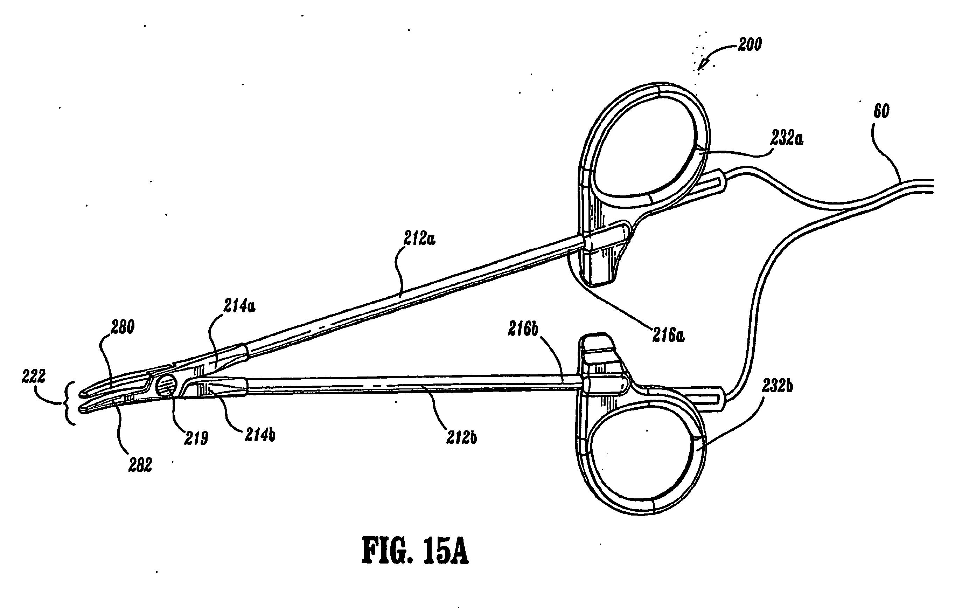

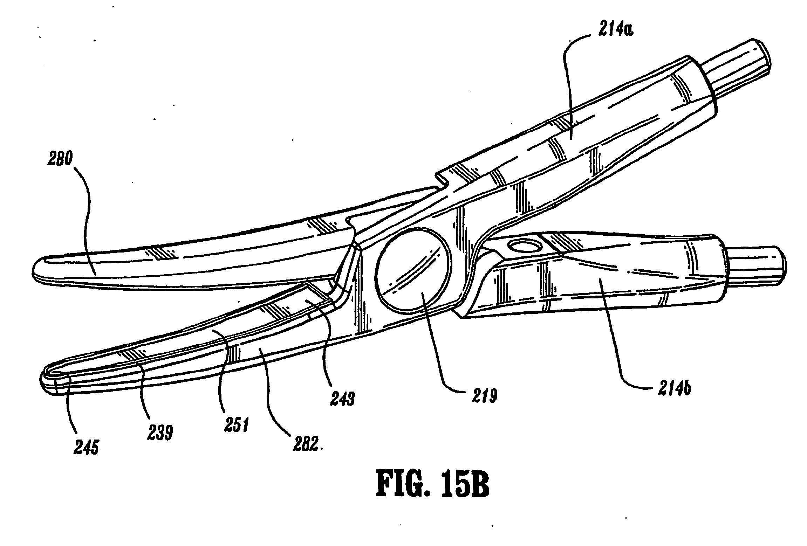

Referring now to Figs. 1-3, one embodiment of bipolar forceps 10 is shown for use with endoscopic surgical procedures and includes a drive rod assembly 11 which is coupled to a handle assembly 18. The drive rod assembly 11 includes an elongated hollow shaft portion 12 having a proximal end 16 and a distal end 14. In the drawings and in the descriptions which follow, the term "proximal", as is traditional, will refer to the end of the bipolar forceps 10 which is closer to the user, while the term "distal" will refer to the end which is further from the user. In addition, although the majority of the figures, i.e., Figs 1-14, show one embodiment of the presently described instrument for use with endoscopic surgical procedures, e.g., forceps 10, it is envisioned that the same inventive concepts as shown and described herein may also be employed with or incorporated on an open surgical instrument 100 such as the embodiment shown by way of example in Figs. 15A and 15B.

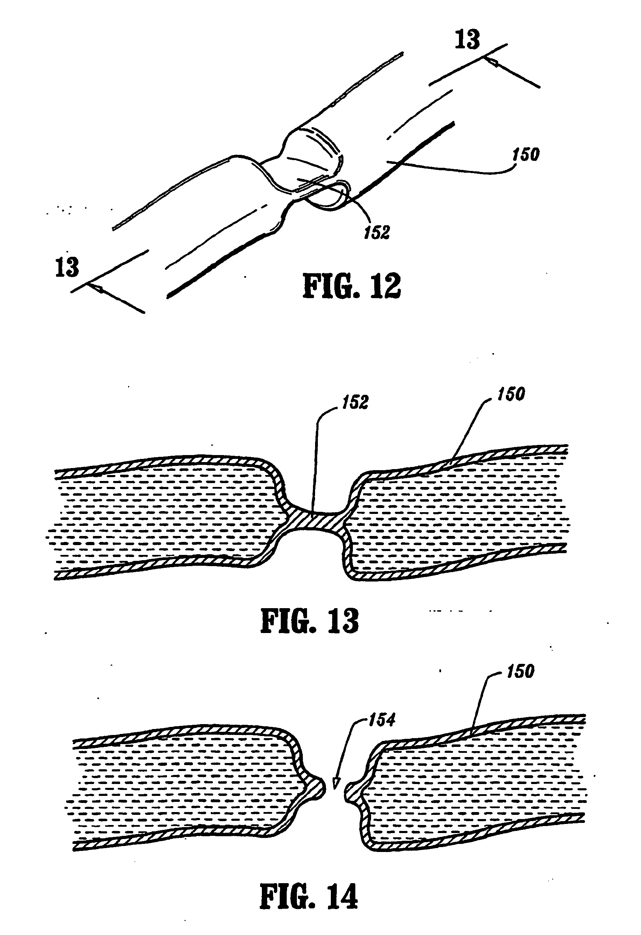

An end effector assembly 22 is attached to the distal end 14 of shaft 12 and includes a pair of opposing jaw members 80 and 82. Preferably, handle assembly 18 is attached to the proximal end 16 of shaft 12 and includes an activator 20 which imparts movement of the jaw members 80 and 82 from an open position wherein the jaw members 80 and 82 are disposed in spaced relation relative to one another, to a clamping or dosed position wherein the jaw members 80 and 82 cooperate to grasp tissue 150 (Fig. 12) therebetween.

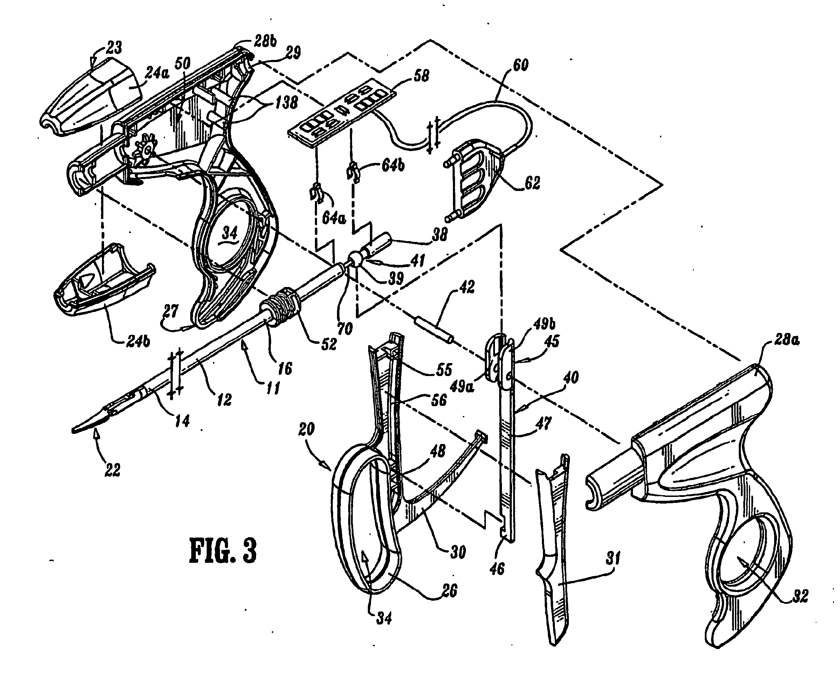

As best seen in Fig. 3, activator 20 includes a movable handle 26 having an aperture 34 defined therethrough for receiving at least one of the operator's fingers and a fixed handle 28 having an aperture 32 defined therethrough for receiving an operator's thumb. Movable handle 26 is selectively moveable from a first position relative to fixed handle 28 to a second position in closer proximity to the fixed handle 28 to close jaw members 80 and 82. Preferably, fixed handle 28 includes a channel 27 which extends proximally for receiving a ratchet 30 which is coupled to movable handle 26. Ratchet 30 allows a user to selectively, progressively and incrementally move jaw members 80 and 82 relative to one another from the open to closed positions. As can be appreciated, ratchet 30 also allows a user to lockingly engage the movable handle 26 and, therefore, jaw members 80, 82 at incremental positions relative to one another prior to and/or during activation. In some cases it may be preferable to include other mechanisms to control and/or limit the movement of handle 26 relative to handle 28 and jaw members 80 and 82 such as, e.g., hydraulic, semi-hydraulic and/or gearing systems.

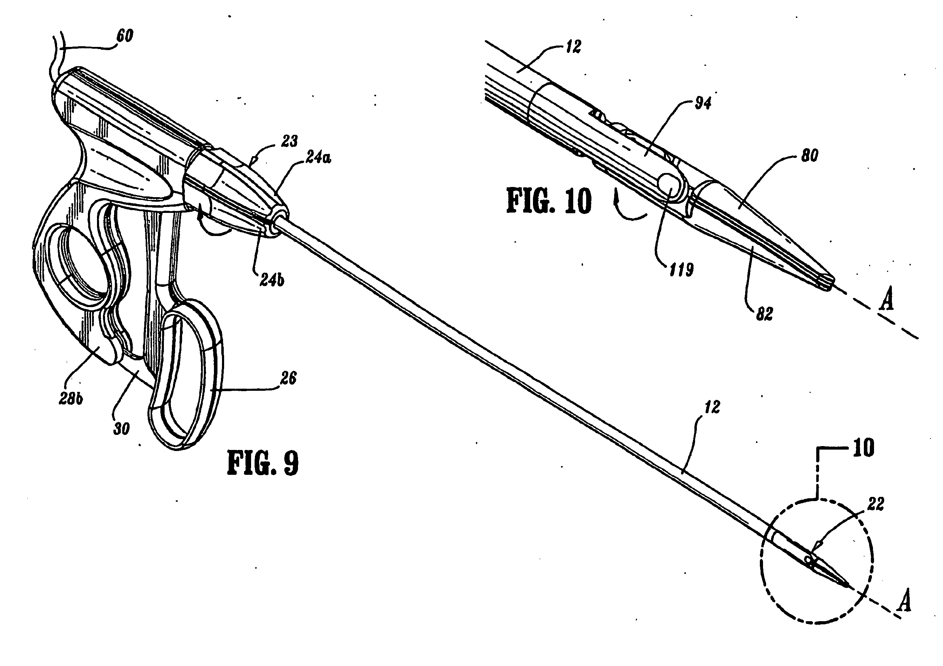

Fixed handle 28 includes a rotating assembly 23 for controlling the rotational movement of end effector assembly 22 about a longitudinal axis "A" of the elongated shaft 12 (see Figs. 9 and 10). Preferably, rotating assembly 23 includes upper and lower knob portions 24a and 24b, respectively, which mechanically interface one another to enclose a gear 52 which is attached to shaft 12. Preferably, the ratio of rotation of rotating assembly 23 to end effector assembly 22 is 1:1, however, it is contemplated that a different gearing structure may be incorporated to increase or decrease the rotational ratio depending upon a particular purpose, e.g., worm gears, gear trains, etc.

Preferably, a pair of handle sections 28a and 28b engage one another by way of a plurality of mechanical interfaces to form fixed handle 28. The mechanical interfaces include sockets 138 which are formed in handle section 28b and which are dimensioned to receive a complementary plurality of detents (not shown) attached to handle section 28a. While the term socket is used herein, it is contemplated that either a male or female mechanical interface may be used on either handle section e.g., 28a with a mating mechanical interface disposed on the opposite handle section, e.g., 28b.

As best seen in Fig. 3, each handle section 28a and 28b is generally hollow such that a cavity 50 is formed therein for housing the various internal-working components which make up the forceps 10. For example, cavity 50 houses a PC board 58 which transfers electrosurgical energy transmitted from an electrosurgical generator (not shown) to each jaw member 80 and 82. A plug 62 connects to the electrosurgical generator and transmits electrosurgical energy to the PC board via a cable 60 which is fed to forceps 10 through a wire port 29 disposed in the proximal end of handle assembly 28.

Preferably, a lost motion mechanism is positioned between each of the handle sections 28a and 28b for maintaining a predetermined or maximum clamping force for sealing tissue between the jaw members 80 and 82. In the particular embodiment shown in Fig. 3, the lost motion mechanism comprises a resilient arm 40 which is connected between handle sections 28a and 28b by pin 42. More particularly, the arm 40 includes a lower end 46, an upper end 45 and shaft portion 47 located therebetween. Preferably, upper end 45 is bifurcated and forms a clevis having upwardly extending flanges 49a and 49b, respectively. Lower end 46 is dimensioned to engage a step-like interface 48 located on movable handle portion 26. The shaft portion 47 is seated within a pivot slot 55 located towards the upper end of handle 26 such that the shaft portion 47 is housed within an elongated channel 56 formed within movable handle portion 26. Preferably, a cover plate 31 attaches to movable handle 26 by conventional means, e.g., snap-fit engagement to secure arm 40 within handle 26.

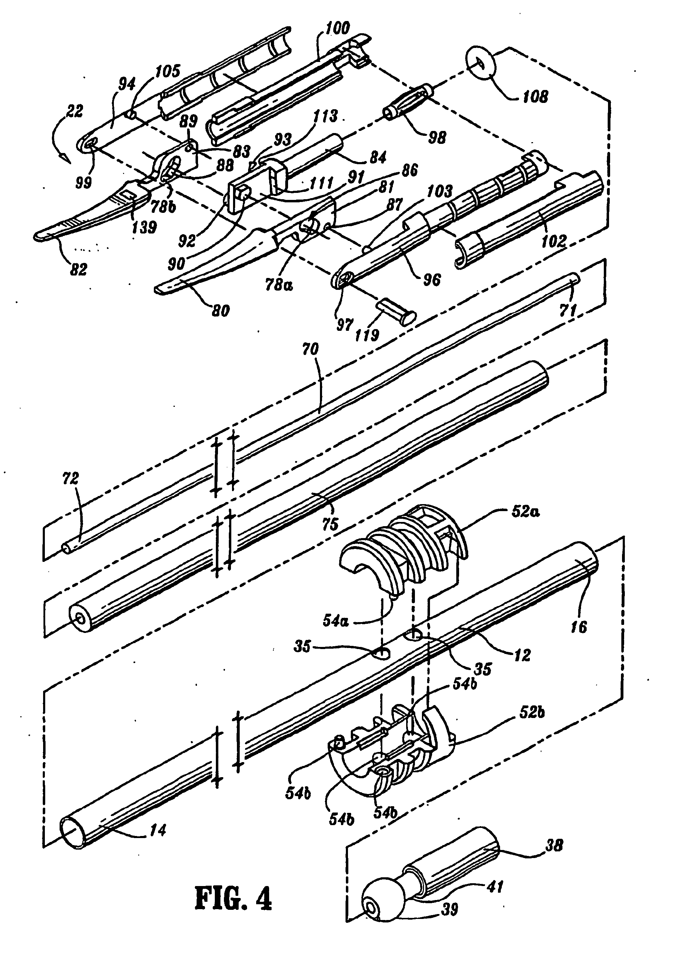

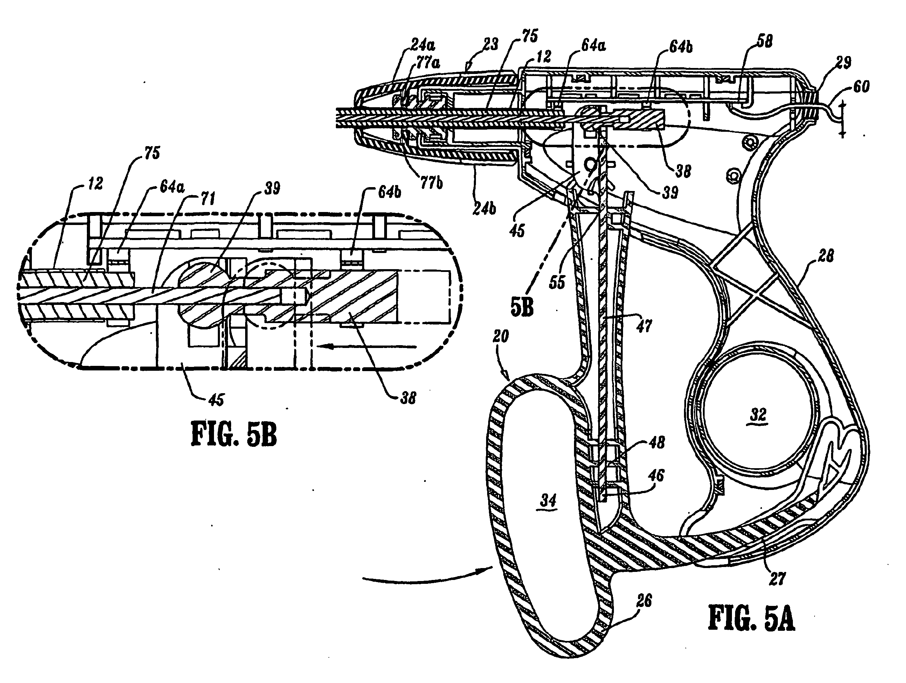

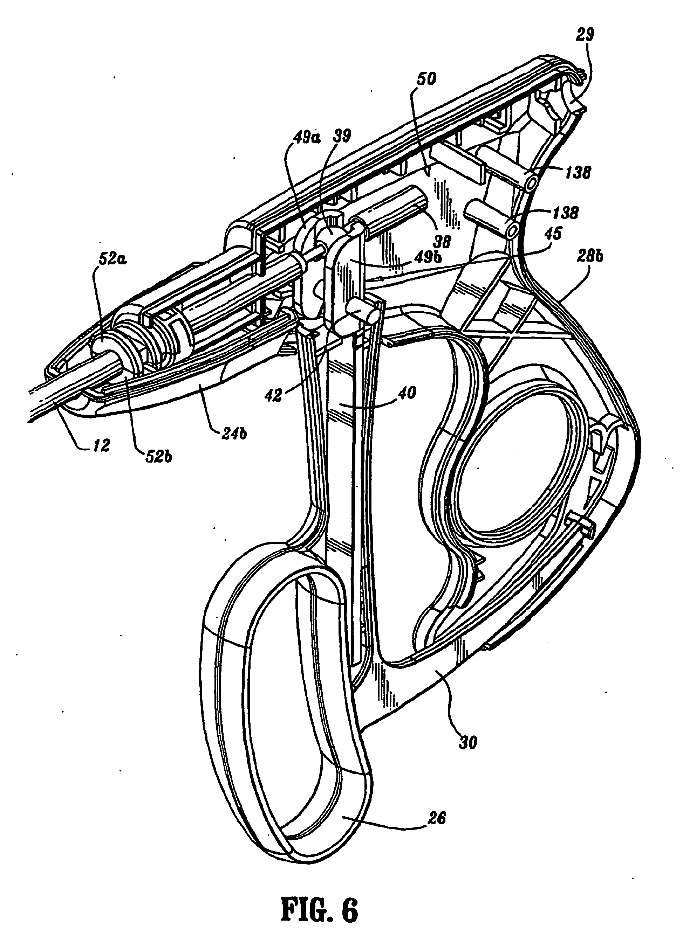

Referring to Fig. 4, rod assembly 11 includes a drive rod 70 which has a proximal end 71 and a distal end 72. A ball contact 38 is attached to the proximal end 71 of drive rod 70 and includes a generally rounded head portion 39 and a notch 41 located between the head portion 39 and the proximal end of ball contact 38. Preferably, clevis flanges 49a and 49b of arm 40 are dimensioned to receive head 39 therebetween when arm 40 is assembled between handle sections 28a and 28b (see Fig. 6). Movement of the handle 26 towards fixed handle 28 imparts pivotal movement of the upper end 45 of arm 40 at pivot slot 55 (see Figs. 5A) which, in turn, imparts movement of the ball contact 38 from a first position wherein the ball contact 38 is disposed further from end effector assembly 22 to a second position wherein ball contact 38 is in closer proximity to end effector assembly 22 (see Fig. 5B). As explained in greater detail below, movement of the ball contact 38 between first and second positions imparts linear movement to drive rod 70 which, in turn, moves jaw members 80 and 82 toward and away from each other.

As can be appreciated by the present disclosure, seating the generally rounded head 39 between clevis flanges 49a and 49b enables the user to utilize the rotating assembly 23 effectively without interfering with the linear movement of the ball contact 38.

As best seen in the exploded view of Fig. 4, the end effector assembly 22 includes first jaw 80, second jaw 82 and an electrically insulating yoke 84 disposed therebetween. Preferably, jaw member 80 and jaw member 82 are movable from the open position to the closed position by movement of the handle assembly 18 as described above. It is also contemplated that either one or both of the jaw members 80 and 82 can be movable relative to one another in the same or similar manner as described above. First jaw member 80 includes a first flange 81 which extends therefrom and a cam slot 86 located therethrough. Likewise, second jaw 82 includes a second flange 83 which extends therefrom and a cam slot 88 located therethrough. Preferably, each jaw 80 and 82 is formed from a stainless steel or some other electrically conductive material.

The end effector assembly 22 also includes an outer nose portion 94 and an inner nose portion 96 which engage jaw members 82 and 80, respectively. A first pivot 105 is located on outer nose portion 94 and is dimensioned to engage a corresponding pivot hole 89 located on flange 83. A second pivot 103 is located on inner nose portion 96 and is dimensioned to engage a corresponding pivot hole 87 located on flange 81. The center of rotation for first jaw member 80 is about a first pivot hole 87 and the center of rotation for second jaw member 82 is about a second pivot hole 89. Preferably, each nose portion 94 and 96 is made from an electrically conductive material and transmits electrosurgical energy to a respective jaw member 82 and 80 as described in more detail below.

As mentioned above with respect to Fig. 3, electrosurgical energy is transmitted from the electrosurgical generator to the PC board 58 which transfers the energy into first and second poles. A pair of terminal clips 64a and 64b are connected to PC board 58 and transfer the first and second poles of alternating potential, respectively, to the different portions of the drive rod assembly 11, i.e., clip 64a connects to shaft 12 and conducts the first pole to jaw member 82 and clip 64b connects to ball contact 38 which connects the second pole to jaw member 80. Since both the drive rod 70 and the shaft 12 are made from an electrically conductive material, an insulation sleeve 75 is disposed between drive rod 70 and shaft 12 to prevent the forceps 10 from short circuiting.

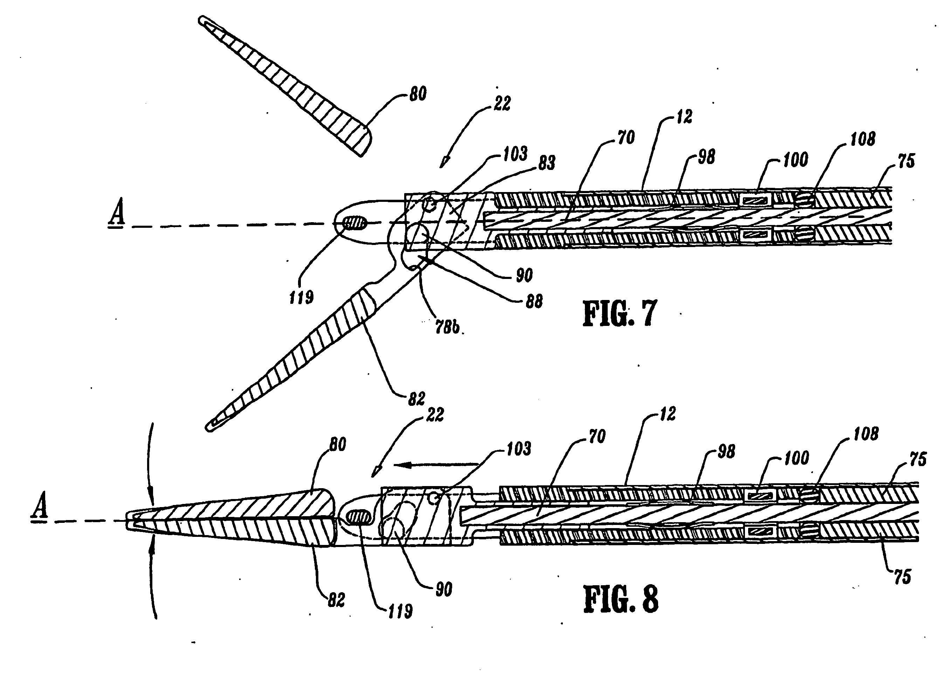

As best seen in Fig. 4, the inner nose portion 96 is electrically connected with drive rod 70 and the outer nose portion 94 is electrically connected to shaft 12. The inner and outer nose portions 96 and 94 capture yoke 84 along with flanges 83 and 81. Yoke 84 moves axially along axis "A" (see Figs. 7 and 8) in a space between inner and outer portions 96 and 94 and a spacer stake 119 maintains the separation of the nose portions 96 and 94 at their distal ends. Stake 119 is dimensioned to engage and lock the inner and outer nose portions 96 and 94 together, which, in turn locks jaw member 80 and 82 atop yoke 84. In some cases it may be preferable to dimension stake 119 such that stake 119 acts as a stop member and/or an additional stop member which controls the gap distance between the opposing jaw members 80 and 82 relative to one another. In this case, stake 119 is formed from an electrically insulative material such as plastic. The nose portions 94 and 96 provide lateral support for the flanges 81 and 83 and help ensure that detents 90 and 92 remain within cam slots 86 and 88, respectively.

End effector assembly 22 also includes an inner insulator 102 and an outer insulator 100 for maintaining electrical isolation between the first and second poles. Outer insulator 100 insulates outer nose portion 94 from inner nose portion 96 and drive rod 70 which conduct the second pole of electrical energy. Inner insulator 102 insulates inner nose portion 96 from outer nose portion 94 and shaft 12 which conduct the first pole of electrical energy. In this manner, outer nose portion 94 can provide electrical continuity between shaft 12 and jaw member 82, while inner nose portion 96 can provide electrical continuity between drive rod 70 and jaw member 80.

Preferably, a spring contact 98 is utilized to maintain the electrical connection between drive rod 70 and inner nose portion 96 during axial motion of the drive rod 70. A donut-shaped spacer 108 can also be utilized as a seal. Sleeve 75 also acts as an insulation between drive rod 70 and shaft 12 and is envisioned to prevent accidental short circuiting of the forceps 10 during movement of the drive rod 70.

As mentioned above and as best seen in Fig. 4, drive rod assembly 11 also includes gear 52 which attaches to shaft 12 and is designed to facilitate rotational movement of the end effector assembly 22 about axis "A". More particularly, gear 52 includes an upper portion 52a and a lower portion 52b which each have a pair of outwardly extending mechanical interfaces 54a and 54b, respectively, which are dimensioned to releasably engage a corresponding pair of mechanical interfaces 35 disposed on shaft 12. Preferably, gear 52 is made from an electrically insulative material such as, e.g., plastic, to avoid transferring electrosurgical energy to the rotating assembly 23. As best seen in Fig. 5A, rotating assembly 23 includes two half sections 24a and 24b which each include a flange 77a and 77b, respectively, which extends outwardly therefrom for engaging gear 52. Rotation of assembly 23 effects rotational movement of the shaft 12 which, in turn, rotates the end effector assembly 22 about axis "A" (see Figs. 9 and 10).

Referring back to Fig. 4, yoke 84 is preferably formed from an electrically insulative material such as plastic. A first side 91 of yoke 84 faces first flange 81 and a second side 93 of yoke 84 faces second flange 83. When yoke 84 is positioned between flanges 81 and 83, yoke 84 electrically insulates and isolates the first jaw member 80 from second jaw member 82. In this manner, bipolar electrosurgical current can be conducted through tissue 150 which is grasped between jaws 80 and 82 without flanges 81 and 83 short circuiting.

Yoke 84 also includes first detent 90 located on the first side 91 which is dimensioned to movably engage cam slot 86 and a second detent 92 located on the second side 93 which is dimensioned to engage cam slot 88. Preferably, the detent and cam slot combination, 90, 86 and 92, 88, respectively, work together as a cam-follower mechanical linkage. Linear motion of drive rod 70 along axis "A" moves the yoke 84 causing detents 90 and 92 to slide within their respective cam slots 86 and 88. In one embodiment, slots 86 and 88 are angled with respect to the distal ends of the jaws 80 and 82 such that the jaws 80 and 82 move in a generally arcuate fashion toward and away from each other.

In another embodiment, the inner periphery of the cam slots 86 and 88 are shaped to include two angles which, in turn, cause the jaw members 80 and 82 to move in two separate and distinct fashions relative to one another upon full extension of the drive rod 70. For example, cam slots 86 and 88 can include a first or proximal stage which effects generally arcuate movement of the jaw members 80 and 82 relative to one another and a second or distal stage wherein the jaw members 80 and 82 move in a more linear fashion relative to one another. It is envisioned that the cam slots 86 and 88 can be dimensioned to effect other movements of the jaw members 80 and 82 relative to one another depending upon a particular purpose, e.g., parabolic movement, cycloidal movement, and/or sinusoidal movement.

As seen best with respect to Figs. 7 and 8, detents 90 and 92 provide a force against the corresponding inner periphery of cam slots 86 and 88 creating a moment about pivots 103 and 105, respectively. Preferably, cam slots 86 and 88 are arranged such that distal motion of the drive rod 70 causes the jaw members 80 and 82 to move together. Once the jaw members 80 and 82 are closed together, it is envisioned that jaws 80 and 82 are held in clamped positioned by a continued compressive force on the rod 70 due to handle member 26. As mentioned above, the handle assembly 18 can include a lost motion mechanism for maintaining a predetermined or maximum clamping force for sealing tissue 150 between the jaw members 80 and 82

One of the advantages of the present disclosure is that excessive clamping forces which are normally associated with detents 90 and 92 are offloaded by the unique configuration of yoke 84 which prevents mechanical failure of the forceps 10. More particularly, the cam slots 86 and 88 are preferably dimensioned such that the cam-follower motion of the detents 90 and 92 within cam slots 86 and 88 simply operate to clamp the tissue 150 between the jaw members 80 and 82 and a small moment arm is created between the detents 90 and 92 and pivots 103 and 105, respectively. Before the detents 90 and 92 reach their distal most positions within the cam slots 86 and 88, respectively, a pair of shoulders 111 and 113 located on the yoke 84 are dimensioned to engage flanges 81 and 83 and offload any additional clamping force applied by the handle assembly 18.

In some cases it may be preferable to dimension cam slots 86 and 88 to have an enlarged distal end or cul-de-sac 78a and 78b, respectively, such that the cam-follower motion of detents 90 and 92 at their distal most point within slots 86 and 88 will come to rest within the cul-de-sac 78a and 78b allowing the closure force to be offloaded by shoulders 111 and 113 abutting flanges 81 and 83. It is envisioned that the cul-de-sacs 78a and 78b which are positioned within cam slots 86 and 88 will relieve shear stress on the detents 90 and 92 approximately at the same time when the shoulder portions 111 and 113 of the yoke 84 engage the flanges 81 and 83 to provide a closure force between the jaw members 80 and 82.

The shoulders 111 and 113 abut the proximal end of flanges 81 and 83 to cause jaw members 80 and 82 to close together with greater dosure force. In other words, shoulder portions 111 and 113 provide a relatively large moment about pivots 103 and 105 to effect a high closure force between the jaw members 80 and 82. The unique configuration of the cam-follower linkage together with the shoulders 111 and 113 offloading high clamping forces prevent detents 90 and 92 from breaking due to mechanical failure. Since the pivots 103 and 105 are preferably made of metal and can withstand relatively high shear forces, the yoke 84 and its component parts can be formed from an insulating material such as plastic without risk of mechanical failure due to the high clamping forces necessary to seal tissue. As mentioned above, forming the yoke 84 from insulative materials will also prevent the jaw members 80 and 82 from shorting.

A mentioned above, two mechanical factors play an important role in determining the resulting thickness of the sealed tissue and effectiveness of the seal, i.e., the pressure applied between opposing jaw members 80 and 82 and the gap between the opposing jaw members 80 and 82 during the sealing process. However, thickness of the resulting tissue seal cannot be adequately controlled by force alone. In other words, too much force and the two jaw members 80 and 82 would touch and possibly short resulting in little energy traveling through the tissue thus resulting in a bad seal. Too little force and the seal would be too thick.

Applying the correct force is also important for other reasons: to oppose the walls of the vessel; to reduce the tissue impedance to a low enough value that allows enough current through the tissue; and to overcome the forces of expansion during tissue heating in addition to contributing towards creating the required end tissue thickness which is an indication of a good seal.

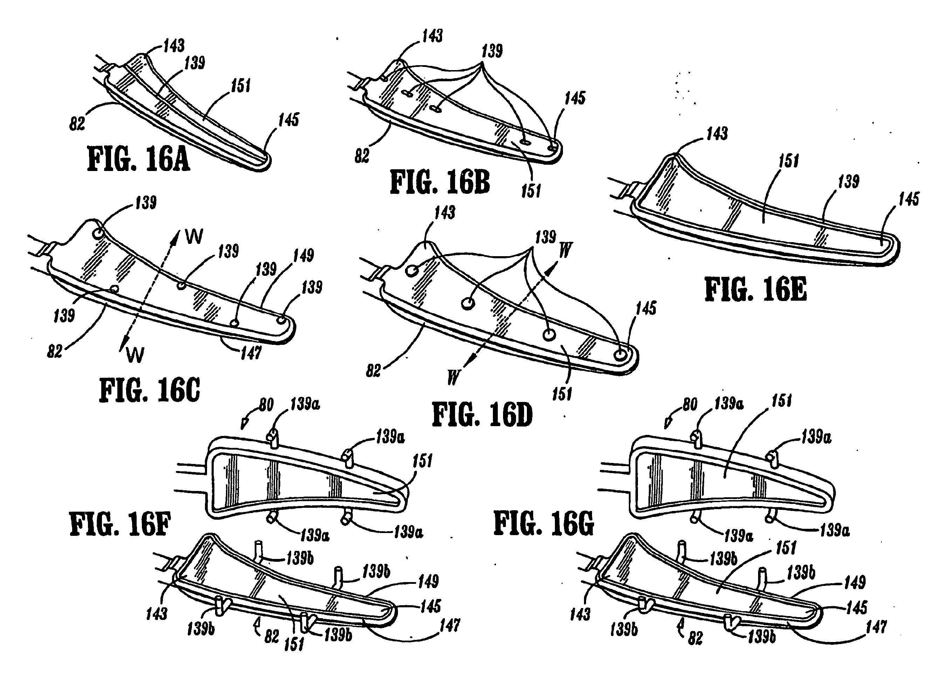

Preferably, the seal surfaces or tissue contacting surfaces 151, 251 (See Figs. 15B and 16A-16G) of the jaw members 80 and 82 are relatively flat to avoid current concentrations at sharp edges and to avoid arcing between high points. In addition and due to the reaction force of the tissue 150 when engaged, jaw members 80 and 82 are preferably manufactured to resist bending. For example and as best seen in Figs. 2 and 16A-16G, the jaw members 80 and 82 are preferably tapered along width "W" which is advantageous for two reasons: 1) the taper will apply constant pressure for a constant tissue thickness at parallel; 2) the thicker proximal portion of the jaw members 80 and 82 will resist bending due to the reaction force of the tissue 150.

As best seen in Fig. 4, in order to achieve a desired gap range (e.g., about 25.4 µm (about 0.001 inches) to about 127 µm (about 0.005 inches) and preferably about 50.8 µm (about 0.002 inches) to about 76.2 µm (about 0.003 inches) and apply a desired force to seal the tissue, at least one jaw member 80 and/or 82 includes a stop member 139 which limits the movement of the two opposing jaw members 80 and 82 relative to one another. Preferably, stop member 139 extends from the sealing surface or tissue contacting surface 151 a predetermined distance according to the specific material properties (e.g., compressive strength, thermal expansion, etc.) to yield a consistent and accurate gap distance during sealing.

As explained above, in some cases it may be preferable to dimension stake 119 such that it acts like a stop member and/or an additional stop member and also controls/limits the movement of the two opposing jaw members 80 and 82 relative to one another. Preferably, stop member 139 and/or stake 119 is made from an insulative material, e.g., parylene, nylon and/or ceramic and is dimensioned to limit opposing movement of the jaw members 80 and 82 to within the above gap range.

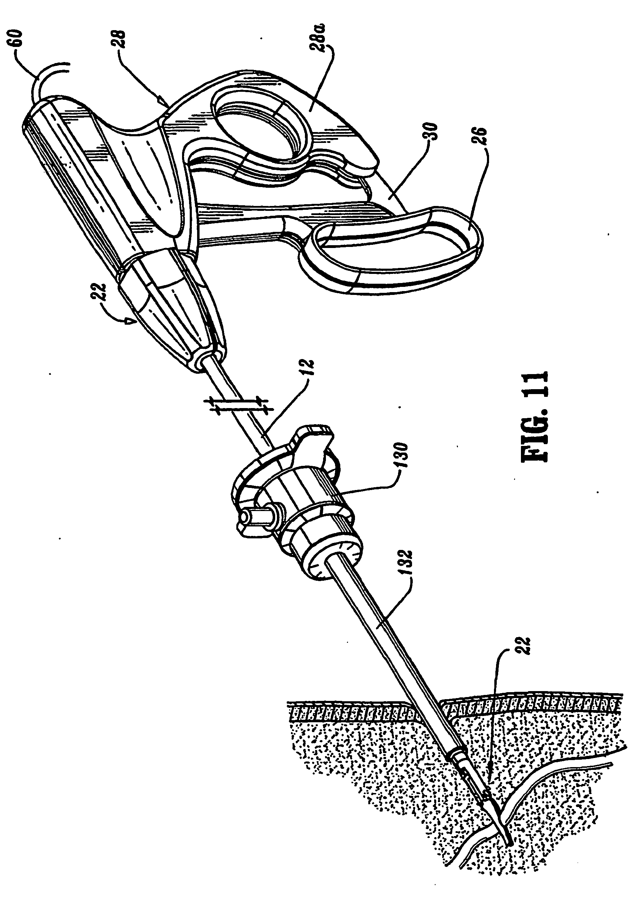

Fig. 11 shows the endoscopic bipolar forceps 10 according to the present disclosure during use wherein movement of the handle assembly applies clamping force on the tubular tissue 150 to effect a seal 152 as shown in Figs. 12 and 13. More particularly, shaft 12 and end effector assembly 22 are inserted through a trocar 130 and cannula 132 and handle 26 is moved progressively towards fixed handle 28 to cause jaw members 80 and 82 to grasp tubular vessel 150 therebetween. After the jaw members 80 and 82 are closed about the tissue 150, the user then applies electrosurgical energy to the tissue 150. By controlling the intensity, frequency and duration of the electrosurgical energy applied to the tissue 150, the user can either cauterize, coagulate/desiccate, seal and/or simply reduce or slow bleeding. As shown in Figs. 13 and 14, once the tubular vessel is sealed, the vessel 150 can be cut along seal 152 to separate the tissue 150 and form gap 154 therebetween.

Fig. 15A and 15B shows an open vessel bipolar surgical forceps 200 according to the present of disclosure. As can be appreciated, forceps 200 also includes an end effector assembly 222 which is attached to respective distal ends 214a and 214b of opposing elongated shafts 212a and 212b. End effector assembly 222 includes jaw members 280 and 282 disposed at distal ends 214a, 214b, respectively, which reside in opposing relation relative to one another and pivot about pivot 219. Preferably, a finger ring 232a and 232b is attached to a corresponding proximal end 216a, 216b of each shaft 212a, 212b, respectively, such that movement of the finger rings 232a, 232b imparts movement of the jaw members 280, 282 relative to one another from an open position (wherein the jaw members 280 and 282 are disposed in spaced relation relative to one another) to a clamping or closed position (wherein jaw members 280 and 282 cooperate to grasp tissue 150 (Fig. 12) therebetween).

Fig. 15B shows an enlarged view of one contemplated configuration of the jaw members 280 and 282 having a stop member 239 which is designed as a raised lip which extends along a the peripheral edge of jaw member 282 from a proximal end 243 to a distal end 245 of the jaw member 282. It is envisioned that forceps 200 can also include any of the envisioned stop member 239 configurations described below with respect to Figs. 16A-16G of which FIGS. 16A to 16G only Figures 16A and 16E are embodiments of the claimed invention.

Figs. 16A-16G of which FIGS. 16A to 16G only Figures 16A and 16E are embodiments of the claimed invention, show various contemplated configurations of the non-conductive stop member 139 disposed on, along or protruding through the bottom jaw member 82 (282). It is envisioned that one or more stop members 139 can be positioned on either or both jaw members 80, 82 (280, 282) depending upon a particular purpose or to achieve a desired result. As can be appreciated by the present disclosure, the various configurations of the stop member 139 are designed to both limit the movement of the tissue 150 prior to and during activation and prevent short circuiting of the jaw members 80, 82 (280, 282) as the tissue 150 is being compressed.

Fig. 16A shows stop member 139 configured as a longitudinal ridge extending from a proximal end 143 to a distal end 145 of jaw member 82. Fig. 16B shows a series of stop members configured as longitudinally-oriented detents extending from the proximal end 143 to the distal end 145 of jaw member 82. Fig. 16C shows a series of circle-like stop members 139 extending from the proximal end 143 to the distal end 145 of jaw member 82 in an alternating, laterally-offset manner relative to one another. It is envisioned that circle-like stop members 139 are positioned proximate the right and left side edges 147, 149, respectively, of jaw member 82 and are substantially equal in size. However, it is envisioned that one or more of the stop members 139 may be dimensioned or shaped differently than the other stop members depending upon a particular purpose or to achieve a desired result.

Fig. 16D also shows a series of circle-like stop members 139 extending from the proximal end 143 to the distal end 145 of the jaw member 82, however, each of the stop members 139 is centrally disposed along width "W" of the jaw member 82. Fig. 16E shows another configuration wherein the stop member 139 is designed as a raised lip which projects from the outer periphery of the jaw member 82.

Fig. 16F shows yet another configuration which includes a stop member 139b which is L-shaped and extends from the side edge 147, 149 of the jaw member 82 in a catamaran-like fashion. Preferably a complimentary stop member 139a is disposed on the jaw member 80 such that the two stop members 139a and 139b abut one another when the jaws are moved towards the closed position. It is envisioned that configuring the jaw members in this fashion will provide lateral consistency and stabilization across width "W" (See Fig. 16D) of the overall gap distance (between about 25.4 µm (about 0.001 inches) to about 127 µm (about 0.005 inches) and preferably about 50.8 µm (about 0.002 inches) to about 76.2 µm (about 0.003 inches)) between the sealing surfaces 151 of opposing jaw members 80, 82 (280, 282). Fig. 16G shows yet another embodiment wherein the stop members 139a and 139b are generally C-shaped.

Preferably, the non-conductive stop member(s) 139 (239) is molded onto the jaw members 80 and 82 (e.g., overmolding, injection molding, etc.), stamped onto the jaw members 80 and 82 or deposited (e.g., deposition) onto the jaw members 80 and 82. For example, one technique involves thermally spraying a ceramic material onto the surface of the jaw member 80 and 82 to form the stop member(s) 139. Several thermal spraying techniques are contemplated which involve depositing a broad range of heat resistant and insulative materials on the electrically conductive surfaces to create stop members 139, e.g., High velocity Oxy-fuel deposition, plasma deposition, etc. Other techniques for disposing the stop member(s) 139 on the electrically conductive surfaces are also contemplated, e.g., slide-on, snap-on, adhesives, molds, etc.

It is envisioned that the stop member 139 (239) protrudes about 25.4 µm (about 0.001 inches) to about 127 µm (about 0.005 inches) from the inner-facing surface of jaw member 82 (282) which, as can be appreciated by the present disclosure, both reduces the possibility of short circuiting between electrodes and enhances the gripping characteristics of the jaw members 80, 82 (280, 282). Preferably, the stop member 139, 239 protrudes about 50.8 µm (about 0.002 inches) to about 76.2 µm (about 0.003 inches) which has been determined to yield an ideal gap distance for producing effective, uniform and consistent tissue seals.

Alternatively, the stop member 139 (239) can be molded onto the inner-facing surface of one or both jaw members 80, 82 (280, 282), or, in some cases, it may be preferable to adhere the stop member 139 (239) to the inner facing surface of one or both of the jaw members 80, 82 by any known method of adhesion. Stamping is defined herein to encompass virtually any press operation known in the trade, including but not limited to: blanking, shearing, hot or cold forming, drawing, bending, and coining.

Figs. 16A-16G of which Figs. 16A to 16G only Figures 16A and 16E are embodiments of the claimed invention show some of the possible configurations of the stop member 139, however, these configurations are shown by way of example and should not be construed as limiting. Other configurations are also contemplated. For example, one or more of the configurations of Figs. 16A-16G may be combined to form a different stop member 139 (239) configuration on the inner-facing surface of one or both of the jaw members 80, 82 (280, 282). Although Figs. 16C and 16D depict circle-like stop members 139 (239) arranged in different configurations on or along jaw member 82 (282), it is contemplated that other shapes may be equally effective in reducing the possibility of short circuiting between electrodes and enhancing tissue grip.

Further, although it is preferable that the stop member 139 (239) protrudes about 25.4 µm (about 0.001 inches) to about 127 µm (about 0.005 inches) and preferably about 50.8 µm (about 0.002 inches) to about 76.2 µm (about 0.003 inches) from the inner-facing surface of the jaw member(s), in some cases it may be preferable to have the stop member 139 (239) protrude more or less depending upon a particular purpose. For example, it is contemplated that the type of material used for the stop member 139 and that material's ability to absorb the large compressive closure forces between jaw members while reducing the possibility of short circuiting between jaw members will vary and, therefore, the overall dimensions of the stop member 139 may vary as well to produce the desired gap distance. In other words, the compressive strength of the material along with the desired or ultimate gap distance required (desirable) for effective sealing are parameters which are carefully considered when forming the stop members 139 (239).

As can be appreciated, one material may have to be dimensioned differently from another material to achieve the same gap distance or desired result. For example, the compressive strength of nylon is different from ceramic and, therefore, the nylon material may have to be dimensioned differently, e.g., thicker, to counteract the closing force of the opposing jaw members and to achieve the same desired gap distance.

From the foregoing and with reference to the various figure drawings, those skilled in the art will appreciate that certain modifications can also be made to the present disclosure without departing from the scope of the present disclosure. For example, it may be preferable to add other features to the forceps 10 (200), e.g., an articulating assembly to axially displace the end effector assembly 22 (222) relative to the elongated shaft 12 (212).

Moreover, it is envisioned that the presently enclosed stop member configurations may also be incorporated onto a disposable and/or partially disposable electrosurgical instrument such as those described in commonly-assigned, pending U.S. Application Serial No. 09/425,696 filed on October 22, 1999 entitled "OPEN VESSEL SEALING FORCEPS WITH DISPOSABLE ELECTRODES" by Tetzlaff et al., U.S. Application Serial No. 09/178,027 filed on October 23, 1998 entitled "OPEN VESSEL SEALING FORCEPS WITH DISPOSABLE ELECTRODES" by Tetzlaff et al. and U.S. Application Serial No. 09/387,883 filed on September 1, 1999 entitled "BIPOLAR ELECTROSURGICAL INSTRUMENT WITH REPLACEABLE ELECTRODES" by Schmaltz et al.

More particularly, it is contemplated that the presently disclosed forceps may include a disposable electrode assembly which is selectively engageable with at least one portion of the electrosurgical instrument, e.g., end effectors, shaft(s) and/or handle(s).

## Claims

1. A bipolar forceps (200), comprising:
at least one elongated shaft (212a, 212b) having opposing jaw members (280, 282) at a distal end (214a, 214b) thereof, the jaw members being movable relative to one another from a first position wherein the jaw members are disposed in spaced relation relative to one another to a second position wherein the jaw members cooperate to grasp tissue (150) therebetween;
a source of electrical energy connected to each jaw member such that the jaw members are capable of conducting energy through tissue held therebetween; and
a stop member (239) disposed as a raised lip along a periphery of an inner facing surface of at least one jaw member and extends from a proximal end (243) to a distal end (245) of the at least one jaw member (282) for controlling the distance between the jaw members when tissue is held therebetween.

2. A bipolar forceps (200), comprising:
at least one elongated shaft (212a, 212b) having opposing jaw members (280, 282) at a distal end (214a, 214b) thereof, the jaw members being movable relative to one another from a first position wherein the jaw members are disposed in spaced relation relative to one another to a second position wherein the jaw members cooperate to grasp tissue (150) therebetween;
a source of electrical energy connected to each jaw member such that the jaw members are capable of conducting energy through tissue held therebetween; and
a stop member which includes a longitudinally oriented ridge which extends from a proximal end to a distal end of an inner facing surface of at least one jaw member for controlling the distance between the jaw members.

## Patentansprüche

1. Bipolare Zange (200), umfassend:
Zumindest einen länglichen Schaft (212a, 212b) mit gegenüberstehenden Backenelementen (280, 282) an einem distalen Ende (214a, 214b) desselben, wobei die Backenelemente relativ zueinander von einer ersten Position, bei der die Backenelemente in einer zueinander beabstandeten Beziehung angeordnet sind, zu einer zweiten Position, bei der die Backenelemente kooperieren, um Gewebe (150) zwischen sich zu ergreifen, beweglich sind;
eine Quelle elektrischer Energie, die mit jedem Backenelement verbunden ist, so dass die Backenelemente in der Lage sind, Energie durch zwischen ihnen gehaltenes Gewebe zu leiten; und
ein Stoppelement (239), das als eine erhöhte Lippe entlang einem Umfang einer nach innen gerichteten Oberfläche von zumindest einem Backenelement angeordnet ist und sich von einem proximalen Ende (243) zu einem distalen Ende (245) von dem zumindest einen Backenelement (282) erstreckt, um den Abstand zwischen den Backenelementen zu steuern, wenn zwischen ihnen Gewebe gehalten wird.

2. Bipolare Zange (200), umfassend:
zumindest einen länglichen Schaft (212a, 212b) mit gegenüberstehenden Backenelementen (280, 282) an einem distalen Ende (214a, 214b) desselben, wobei die Backenelemente relativ zueinander von einer ersten Position, bei der die Backenelemente in einer zueinander beabstandeten Beziehung angeordnet sind, zu einer zweiten Position, bei der die Backenelemente kooperieren, um Gewebe (150) zwischen sich zu ergreifen, beweglich sind;
eine Quelle elektrischer Energie, die mit jedem Backenelement verbunden ist, so dass die Backenelemente in der Lage sind, Energie durch zwischen ihnen gehaltenes Gewebe zu leiten; und
ein Stoppelement, das eine längs ausgerichtete Kante enthält, die sich von einem proximalen Ende zu einem distalen Ende einer nach innen gerichteten Oberfläche von zumindest einem Backenelement zum Steuern des Abstands zwischen den Backenelementen erstreckt.

## Revendications

1. Pince bipolaire (200), comprenant:
au moins un arbre oblong (212a, 212b) ayant des éléments de mâchoire opposés (280, 282) à une extrémité distale (214a, 214b) de celui-ci, les éléments de mâchoire étant déplaçables l'un relativement à l'autre à partir d'une première position où les éléments de mâchoire sont disposés selon une relation espacée l'un par rapport à l'autre à une seconde position où les éléments de mâchoire coopèrent pour saisir le tissu (150) entre eux;
une source d'énergie électrique reliée à chaque élément de mâchoire de façon que les éléments de mâchoire soient aptes à conduire l'énergie à travers le tissu retenu entre eux; et
un élément d'arrêt (239) disposé comme une lèvre relevée le long d'une périphérie d'une surface orientée vers l'intérieur d'au moins un élément de mâchoire et s'étend d'une extrémité proximale (243) à une extrémité distale (245) du au moins un élément de mâchoire (282) pour régler la distance entre les éléments de mâchoire lorsque le tissu est tenu entre ceux-ci.

2. Pince bipolaire (200), comprenant:
au moins un arbre oblong (212a, 212b) ayant des éléments de mâchoire opposés (280, 282) à une extrémité distale (214a, 214b) de ceux-ci, les éléments de mâchoire étant déplaçables l'un relativement à l'autre à partir d'une première position où les éléments de mâchoire sont disposés selon une relation espacée l'un par rapport à l'autre à une seconde position où les éléments de mâchoire coopèrent pour saisir le tissu (150) entre eux;
une source d'énergie électrique reliée à chaque élément de mâchoire de façon que les éléments de mâchoire soient aptes à conduire l'énergie à travers le tissu tenu entre ceux-ci; et
un élément d'arrêt qui comprend une arête orientée longitudinalement qui s'étend d'une extrémité proximale à une extrémité distale d'une surface orientée vers l'intérieur d'au moins un élément de mâchoire pour régler la distance entre les éléments de mâchoire.
